Europäisches Patentamt

European Patent Office

Office européen des brevets

(11) Veröffentlichungsnummer : **0 143 179**
**B1**

# EUROPÄISCHE PATENTSCHRIFT

(45) Veröffentlichungstag der Patentschrift :
**01.10.86**

(21) Anmeldenummer : **84109285.1**

(22) Anmeldetag : **04.08.84**

(51) Int. Cl.⁴ : **C 07 C 53/12, C 07 C 51/42,
C 07 C 51/573**

(54) Verfahren zur Abtrennung von Jod und dessen Verbindungen aus den bei der Carbonylierung von Dimethylether, Methylacetat oder Methanol erhaltenen Carbonylierungsprodukten.

(30) Priorität : **01.09.83 DE 3331548**

(43) Veröffentlichungstag der Anmeldung :
**05.06.85 Patentblatt 85/23**

(45) Bekanntmachung des Hinweises auf die Patenterteilung : **01.10.86 Patentblatt 86/40**

(84) Benannte Vertragsstaaten :
**AT BE CH DE FR GB IT LI NL**

(56) Entgegenhaltungen :
**EP-A- 0 082 349
US-A- 4 246 195**

(73) Patentinhaber : **HOECHST AKTIENGESELLSCHAFT
Postfach 80 03 20
D-6230 Frankfurt am Main 80 (DE)**

(72) Erfinder : **Erpenbach, Heinz, Dr.
Oberbuschweg 22
D-5000 Köln 50 (DE)**
Erfinder : **Gehrmann, Klaus, Dr.
Geschwister-Scholl-Strasse 32
D-5042 Erftstadt (DE)**
Erfinder : **Ohorodnik, Alexander, Dr.
Kastanienweg 24
D-5042 Erftstadt (DE)**
Erfinder : **Lork, Winfried
Siegfried-von-Westerburg-Strasse 14
D-5042 Erftstadt (DE)**
Erfinder : **Joest, Herbert
Heddinghovener Strasse 3
D-5042 Erftstadt (DE)**

## Beschreibung

Die Erfindung betrifft ein Verfahren zur Abtrennung von Jod und dessen Verbindungen aus den bei der Carbonylierung von Dimethylether und/oder Methylacetat und/oder Methanol erhaltenen Carbonylierungsprodukten Essigsäure und/oder Acetanhydrid und/oder Ethylidendiacetat.

Die DE-B-21 04 828 beschreibt ein Verfahren zur Entfernung geringer Halogenidverunreinigungen aus einer Essigsäure, welche vorzugsweise durch Reaktion eines Alkohols oder Olefins mit Kohlenmonoxid in Gegenwart eines Katalysatorsystems aus einer Edelmetall- und einer Halogenkomponente gewonnen wurde, durch Behandlung mit Kaliumpermanganat, Natriumpermanganat, Kaliumdichromat, Natriumdichromat, Chromtrioxid, Chromkaliumoxalat, Kaliumchlorchromat, Kaliumchlorat und/oder Kaliumchromat bei 16 bis 200 °C. Hierbei wird eine weniger als 500 ppb Halogenid, insbesondere Jodid, enthaltende Essigsäure vor oder auch während der Destillation mit einer der genannten Metallverbindungen in einer Menge bis zu 1,0 Gew%, bezogen auf die Essigsäure, in Berührung gebracht, wobei man eine Abtrennung der Halogenidverbindung zwischen 90 und 98 % erreicht. Zur Reinigung Acetanhydrid enthaltender Carbonylierungsprodukte ist dieses Verfahren weitaus weniger wirksam, da die genannten Oxydationsmittel auch mit Acetanhydrid reagieren und das Jodid daher nicht mehr binden können.

Die DE-A-22 56 510 beansprucht ein Verfahren zur Entfernung sehr geringer Mengen von Jod aus Essigsäure, bei dem in einem System aus zwei Destillationskolonnen die jodhaltigen Verbindungen durch Zusatz von Oxiden, Hydroxiden, Carbonaten, Bicarbonaten und Salzen schwacher organischer Säuren von Alkali- und Erdalkalimetallen sowie Gemischen der Alkali- oder Erdalkalimetallverbindungen mit Hypophosphoriger Säure je nach vorhandenem Jodgehalt bis zu einem Endgehalt von < 40 ppb bis < 5 ppb aus der eingesetzten Essigsäure entfernt werden können. Bei diesem Verfahren werden sowohl die Salze der Alkali- und Erdalkalimetalle als auch die Hypophosphorige Säure in Form wäßriger Lösungen eingesetzt. Das bedeutet, daß diese Reinigungsmethode zur Entfernung von verunreinigenden Jodverbindungen aus Essigsäureanhydrid nicht geeignet ist, da hierbei das Anhydrid einer Verseifung unterliegt.

Die DE-A-29 01 359 betrifft ein Verfahren zur Entfernung von Jod aus organischen Verbindungen, bei dem man die Jod enthaltenden organischen Verbindungen bei 50 bis 200 °C mit einem Oxidationsmittel behandelt und das Reaktionsgemisch gleichzeitig oder anschließend mit einem Adsorptionsmittel in Berührung bringt. Als Oxidationsmittel wird bevorzugt Sauerstoff oder Wasserstoffperoxid und als Adsorptionsmittel Aktivkohle verwendet. In den Beispielen wird als Oxidationsmittel Wasserstoffperoxid (20 %ig) angegeben. Die Entfernung des Jods gelingt zu 92,4 bis 99,5 %, wobei der Jodgehalt nur bis auf 40 ppm gesenkt wird, was den Reinheitsansprüchen bei weitem nicht genügt. Auch hier ist von Nachteil, daß durch den Einsatz von Wasserstoffperoxid dem System ebenfalls Wasser zugeführt wird.

Die DE-A-31 07 731 beansprucht ein Verfahren zur Abtrennung organischer Jodverbindungen von Carbonylierungsprodukten des Methanols, Methylacetats und Dimethylethers durch Flüssigphasenextraktion mit einem nichtaromatischen Kohlenwasserstoff. Hierbei werden gemäß den Beispielen die Jodgehalte nur bis auf höchstens 100 ppb Jod herabgesetzt. Abgesehen von den zu hohen Jodwerten erfordert dieses Verfahren wegen der zusätzlichen Destillation des Extraktionsmittels einen wesentlich höheren Aufwand.

Nach der Lehre der DE-A-29 40 751 entfernt man als Verunreinigungen vorhandene Jodverbindungen aus Carbonylierungsprodukten des Methylacetats durch Behandeln mit Cäsium-, Kalium- und/oder Natriumacetat, wobei man die entsprechenden Alkalimetalljodide erhält. Die Methyljodid-Abreicherung auf nur 2 ppm genügt nicht den Anforderungen an ein gereinigtes Carbonylierungsprodukt und dessen Weiterverarbeitung. Im übrigen wird durch die gaschromatographische Analyse auf Methyljodid der Gesamtjodgehalt im Carbonylierungsprodukt nicht vollständig erfaßt, so daß eine Aussage über die wirkliche Jodabtrennung schwer nachvollziehbar ist.

Die vorliegende Erfindung beschreibt nun ein Verfahren, bei dem sich die Entfernung jodhaltiger Substanzen nicht nur auf Alkyl- und Aryljodide oder andere leicht destillierbare jodhaltige Verbindungen beschränkt, sondern bei dem es gelingt, Jod in jeder Art von gebundener wie auch elementarer Form zu fixieren und von dem zu reinigenden Einsatzprodukt verfahrenstechnisch einfach und nahezu quantitativ abzutrennen. Als Beispiele entfernbarer Jodverbindungen seien Methyljodid, Butyljodid, Jodaceton, Jodbenzol, Acetyljodid, Benzoyljodid, Jodwasserstoff sowie gegebenenfalls substituierte Ammonium- und Phosphoniumjodide genannt.

Im einzelnen ist das Verfahren der Erfindung nunmehr dadurch gekennzeichnet, daß man, zur Herabsetzung der Menge des die Carbonylierungsprodukte verunreinigenden Gesamtjods auf Gehalte unterhalb 20 ppb Jod, die Carbonylierungsprodukte bei Temperaturen von 50 bis 200 °C, vorzugsweise 80 bis 140 °C, mit Wasserstoff in Gegenwart mindestens eines Edelmetalls aus der Gruppe VIII des Periodensystems der Elemente behandelt und vom Edelmetall abtrennt.

Das Verfahren der Erfindung ist weiterhin bevorzugt und wahlweise dadurch gekennzeichnet, daß man

a) Carbonylierungsprodukte mit weniger als 100 ppm Gesamtjod einsetzt,

b) das Edelmetall in feiner Verteilung auf einem Träger einsetzt,

c) die Behandlung der Carbonylierungsprodukte während 0,2 bis 6 Stunden vornimmt,

d) die Behandlung der Carbonylierungsprodukte unter einem Druck von 0,5 bis 10 bar vornimmt,

e) bezogen auf die eingesetzten Carbonylierungsprodukte, 0,001 bis 1 Masse% Edelmetall einsetzt,

f) je Mol Carbonylierungsprodukt 0,001 bis 1 mol Wasserstoff einsetzt.

Zur Fixierung der jodhaltigen Verunreinigungen setzt man zweckmäßigerweise dem aus der letzten Reinigungsstufe des gesamten Carbonylierungsprozesses abgezogenen und bisher unbehandelten Produkt das Edelmetall zu, welches bevorzugt auf einem Träger fein verteilt vorliegt. Am Edelmetall des Trägers fixierte jodhaltige Verbindungen werden vorzugsweise durch Abfiltrieren des auf den Träger aufgebrachten Edelmetalls aus dem Reinprodukt abgetrennt. Je nach seinem Beladungsgrad wird das Edelmetall wieder zu erneutem Einsatz rückgeführt oder durch frisches Edelmetall ersetzt.

Selbstverständlich können das zur Eliminierung der jodhaltigen Verunreinigungen erforderliche Edelmetall und der Wasserstoff auch vor der letzten Reinigungsstufe, in der der hochsiedende Rückstand vom Carbonylierungsprodukt entfernt wird, zugesetzt werden.

Nach dem Verfahren der Erfindung kann der Gesamtjodgehalt im behandelten Carbonylierungsprodukt bis unter die Nachweisbarkeitsgrenze von < 5 ppb (= 5 Gewichtsteile Jod pro 1 Milliarde ($10^9$) Gewichtsteile Carbonylierungsprodukt) gesenkt werden.

Die Aufbringung des Edelmetalls auf den Träger ist nicht Gegenstand der Erfindung. Bevorzugt wird das Edelmetall in einer besonders feinverteilten und katalytisch wirksamen Form auf das Trägermaterial aufgebracht, indem der ausgewählte Träger zunächst mit der Edelmetallsalzlösung getränkt und anschließend mit einer ammoniakalischen Hydrazinlösung behandelt wird. Dabei fällt das Edelmetall in sehr feinverteilter Form auf dem Träger aus und ist nach dem Waschen bis zum Neutralpunkt und anschließendem Trocknen einsatzbereit. Auch die Freisetzung des Edelmetalls durch Reduktion mit Wasserstoff bei erhöhter Temperatur ist möglich.

Als Träger für das Edelmetall können u. a. Kieselsäure oder Aluminiumoxid dienen. Die Korngröße des Trägers bewegt sich je nach Einsatzart, z. B. als Fest- oder Wirbelbettkatalysator, in einem weiten Bereich und liegt dementsprechend zwischen 0,01 und 10 mm. Desgleichen umfaßt seine innere Oberfläche (bestimmt nach BET) den Bereich zwischen 1 und 700 $m^2$/g. Die Variationsbreite der Konzentration des auf dem Träger aufgetragenen Edelmetalls ist ebenfalls groß, bevorzugt wird sie jedoch zwischen 0,5 und 5 Masse% gehalten. Die Abtrennung des Carbonylierungsproduktes vom Edelmetall erfolgt vorzugsweise durch Filtration.

Der verwendete Wasserstoff wird bevorzugt in reiner Form eingesetzt. Er kann aber auch durch z. B. Stickstoff, Argon und andere inerte Komponenten verunreinigt sein.

Als Edelmetalle eignen sich Ruthenium, Osmium, Iridium, besonders jedoch Rhodium, Palladium und Platin.

Das Verfahren der Erfindung kann sowohl in diskontinuierlicher als auch kontinuierlicher Betriebsweise durchgeführt werden.

Die analytische Bestimmung des Gesamtjods erfolgt durch die jodkatalysierte Reaktion zwischen Arsen- und Cerionen mit photometrischer Endbestimmung des Cers.

Beispiel 1

200 g (1,96 Mol) Essigsäureanhydrid, verunreinigt mit 1 ppm Gesamtjod, werden mit 0,15 g Palladium, welches auf 10 g Kieselgel (Korngröße 0,01-0,2 mm, BET-Oberfläche 350 $m^2$/g) feinverteilt aufgezogen ist, versetzt und mit 3 l/h Wasserstoff bei einer Temperatur von 100 °C begast. Nach einer Begasungszeit von 3 h (0,20 Mol $H_2$/Mol $Ac_2O$) wird die Wasserstoffzufuhr abgestellt, das Essigsäureanhydrid filtriert und analysiert. Es wird ein Gesamtjodwert von < 5 ppb gefunden, woraus sich eine Jodabtrennung von > 99,5 % errechnet.

Beispiel 2 (Vergleichsbeispiel)

200 g Essigsäureanhydrid, verunreinigt mit 1 ppm Gesamtjod, werden mit 0,15 g Palladium, welches auf 10 g Kieselgel (Korngröße 0,01-0,2 mm, BET-Oberfläche 350 $m^2$/g) feinverteilt aufgezogen ist, versetzt und bei einer Temperatur von 100 °C mit 11 l/h Stickstoff begast. Nach einer Begasungszeit von wiederum 3 h wird die Stickstoffzufuhr beendet und filtriert. Im Filtrat wird ein Gesamtjodwert von 200 ppb gefunden, woraus sich die Jodabtrennung mit nur 80 % ergibt.

Beispiel 3

200 g Essigsäureanhydrid, verunreinigt mit 3 ppm Gesamtjod, werden mit 0,15 g Rhodium, welches auf 10 g Kieselgel (Korngröße 0,01-0,2 mm) feinverteilt aufgezogen ist, versetzt und mit 1 l/h Wasserstoff bei einer Temperatur von 110 °C und einem Druck von 1,5 bar begast. Nach einer Begasungsdauer von 2 h wird die Zufuhr von Wasserstoff (0,044 Mol $H_2$/Mol $Ac_2O$) abgestellt, das Essigsäureanhydrid filtriert und analysiert. Aus dem gefundenen Gesamtjodwert von 14 ppb errechnet sich eine Jodabtrennung von 99,5 %.

Beispiel 4

200 g Essigsäureanhydrid, verunreinigt mit 2 ppm Gesamtjod, werden mit 0,10 g Platin, welches auf 15 g Kieselgel (Korngröße 0,01-0,2 mm) feinverteilt aufgezogen ist, versetzt und mit 0,5 l/h

Wasserstoff bei einer Temperatur von 100 °C 3 h behandelt (0,033 Mol H$_2$/Mol Ac$_2$O). Anschließend wird das Essigsäureanhydrid filtriert und analysiert. Im Filtrat wird ein Gesamtjodwert von 18 ppb ermittelt, woraus sich eine Jodabtrennung von 99,1 % ergibt.

Beispiel 5

Stündlich werden 200 g Essigsäureanhydrid, welches mit 1 ppm Gesamtjod verunreinigt ist, und 0,4 l Wasserstoff (0,009 Mol H$_2$/Mol Ac$_2$O) über 100 ml (= 66 g) eines mit Palladium beaufschlagten Trägers (BET-Oberfläche 190 m$^2$/g) bei einer Temperatur von 90 °C geleitet. Der mit Palladium beaufschlagte Träger besteht aus 3 × 8 mm Aluminiumoxid-Strängen, die mit 1,3 Masse% Pd belegt sind, und ist in einem beheizbaren Rohr fest angeordnet. Nach einer Verweilzeit von 30 Minuten ergibt die Analyse in dem über den Katalysator geleiteten und gesammelten Essigsäureanhydrid einen Gesamtjodwert von 10 ppb. Die Jodabtrennung beträgt somit 99 %.

Beispiel 6

200 g Essigsäureanhydrid, verunreinigt mit 40 ppm Gesamtjod, werden mit 0,15 g Palladium, welches auf 10 g SiO$_2$ (Korngröße 0,01-0,2 mm, BET-Oberfläche 580 m$^2$/g) feinverteilt aufgezogen ist, versetzt und mit 3 l/h Wasserstoff bei einer Temperatur von 120 °C und einem Druck von 1,5 bar begast. Nach einer Begasungszeit von 6 h wird der Wasserstoff abgestellt (0,40 Mol H$_2$/Mol Ac$_2$O), das Essigsäureanhydrid vom auf SiO$_2$ aufgezogenen Palladium abfiltriert und analysiert. Aus dem gefundenen Jodwert von 17 ppb errechnet sich die Jodabtrennung zu > 99,9 %.

**Patentansprüche**

1. Verfahren zur Abtrennung von Jod und dessen Verbindungen aus den bei der Carbonylierung von Dimethylether, Methylacetat oder Methanol erhaltenen Carbonylierungsprodukten Essigsäure, Acetanhydrid oder Ethylidendiacetat, dadurch gekennzeichnet, daß man, zur Herabsetzung der Menge des die Carbonylierungsprodukte verunreinigenden Gesamtjods auf Gehalte unterhalb 20 ppb Jod, die Carbonylierungsprodukte bei Temperaturen von 50 bis 200 °C mit Wasserstoff in Gegenwart mindestens eines Edelmetalls aus der Gruppe VIII des Periodensystems der Elemente behandelt und vom Edelmetall abtrennt.

2. Verfahren nach Anspruch 1, dadurch gekennzeichnet, daß man Carbonylierungsprodukte mit weniger als 100 ppm Gesamtjod einsetzt.

3. Verfahren nach einem der vorhergehenden Ansprüche, dadurch gekennzeichnet, daß man das Edelmetall in feiner Verteilung auf einem Träger einsetzt.

4. Verfahren nach einem der vorhergehenden Ansprüche, dadurch gekennzeichnet, daß man die Behandlung der Carbonylierungsprodukte während 0,2 bis 6 Stunden vornimmt.

5. Verfahren nach einem der vorhergehenden Ansprüche, dadurch gekennzeichnet, daß man die Behandlung der Carbonylierungsprodukte unter einem Druck von 0,5 bis 10 bar vornimmt.

6. Verfahren nach einem der vorhergehenden Ansprüche, dadurch gekennzeichnet, daß man, bezogen auf die eingesetzten Carbonylierungsprodukte, 0,001 bis 1 Masse % Edelmetall einsetzt.

7. Verfahren nach einem der vorhergehenden Ansprüche, dadurch gekennzeichnet, daß man je Mol Carbonylierungsprodukt 0,001 bis 1 mol Wasserstoff einsetzt.

**Claims**

1. Process for separating iodine and its compounds from the carbonylation products acetic acid, acetic anhydride or ethylidene diacetate obtained by subjecting dimethylether, methyl acetate or methanol to carbonylation, characterized in that, in order to reduce the quantity of total iodine contaminating the carbonylation products to less than 20 ppb iodine, the carbonylation products are treated at temperatures of 50 to 200 °C with hydrogen in the presence of at least one noble metal selected from group VIII of the Periodic System of the elements, and separated from the noble metal.

2. Process as claimed in claim 1, wherein carbonylation products containing less than 100 ppm total iodine are used.

3. Process as claimed in claim 1 or 2, wherein the noble metal is finely distributed on a carrier.

4. Process as claimed in any of the preceding claims, wherein the carbonylation products are treated over a period of 0.2 to 6 hours.

5. Process as claimed in any of the preceding claims, wherein the carbonylation products are treated under a pressure of 0.5 to 10 bars.

6. Process as claimed in any of the preceding claims, wherein 0.001 to 1 mass% noble metal, based on the carbonylation products, is used.

7. Process as claimed in any of the preceding claims, wherein 0.001 to 1 mol hydrogen is used per mol carbonylation product.

**Revendications**

1. Procédé de séparation de l'iode et de ses composés des produits de carbonylation, acide acétique, anhydride acétique ou diacétate d'éthylidène, obtenus dans la carbonylation de l'éther diméthylique, de l'acétate de méthyle ou du méthanol, caractérisé en ce que, pour réduire la quantité d'iode total contaminant les produits de carbonylation à des teneurs de moins de 20 ppb, on traite les produits de carbonylation à des températures de 50-200 °C par l'hydrogène en présence d'au moins un métal noble du groupe VIII de la Classification Périodique des éléments et on les sépare du métal noble.

2. Procédé selon la revendication 1, caractérisé en ce que l'on utilise des produits de carbonylation d'une teneur en iode total inférieure à 100 ppm.

3. Procédé selon l'une des revendications précédentes, caractérisé en ce que l'on utilise le métal noble à l'état finement divisé sur un support.

4. Procédé selon l'une des revendications précédentes, caractérisé en ce que l'on traite les produits de carbonylation pendant 0,2-6 heures.

5. Procédé selon l'une des revendications précédentes, caractérisé en ce que l'on traite les produits de carbonylation sous une pression de 0,5-10 bars.

6. Procédé selon l'une des revendications précédentes, caractérisé en ce que l'on utilise le métal noble à raison de 0,001-1 % en masse, par rapport aux produits de carbonylation.

7. Procédé selon l'une des revendications précédentes, caractérisé en ce que l'on utilise 0,001-1 mole d'hydrogène par mole de produit de carbonylation.